# EUROPEAN PATENT APPLICATION

(11) **EP 4 012 583 A1**
(43) Date of publication of application: **15.06.2022**
(21) Application number: 21211968.9
(22) Date of filing: 02.12.2021
(51) Int. Cl.: G06F 16/9538, G06Q 50/06, C12M 1/107

(54) **METHOD, COMPUTER PROGRAM, DATA SYSTEM AND DISTRIBUTION SYSTEM FOR THE DISTRIBUTION OF GASEOUS FUEL FROM A FUEL PRODUCTION FACILITY TO CONSUMERS**

(30) Priority: 11.12.2020 FI 20206280
(71) Applicant: Demeca OY, 86600 Haapavesi (FI)
(72) Inventor: PENNINKANGAS, Lauri, 86600 Haapavesi (FI); VINKKI, Pekka, 86600 Haapavesi (FI)
(74) Representative: Berggren Oy

(57) **Abstract**

In the method, gaseous fuel is distributed from a fuel production facility (50) to consumers from distribution stations (100), which are in connection with the fuel production facility and which distribution stations are at a distance from each other. In the method, the information of several distribution stations in connection with the fuel production facility is stored in a database (12), and the information of said several distribution stations is presented to the consumers on a digital terminal (14). The information to be presented to the consumers comprises at least the location of the fuel distribution station, the price of the fuel to be sold, and/or the amount of fuel on sale. Advantageously, the distribution stations are presented on a digital map, which map can be read on a portable terminal used by the consumer, such as a mobile phone. With the aid of the information shown on the terminal, the consumer can find a suitable distribution station from the ones presented. A computer program is used in the method, which computer program is downloaded onto a terminal used by the consumer.

## Description

### Field of the invention

The invention relates to a method for distribution of gaseous fuel from a fuel production facility to consumers, in which method fuel is distributed from distribution stations, which are in connection with the fuel production facility and which distribution stations are at a distance from each other. The invention additionally relates to a computer program, data system and distribution system for arranging the distribution of a gaseous fuel.

### Prior art

A substantial amount of sludge and biomass from domestic animal excrement is formed on many farms, which can be used for manufacturing biogas in bioreactors. Biogas generated in bioreactors can be utilized on farms e.g. for heating buildings and producing electricity. The amount of biogas formed on farms is often so large that it would suffice also for use outside the farm. The sale and use of biogas outside the farm is however hindered by the absence of a set up sale and distribution network.

A biogas distribution station can be built in connection to the biogas facility, from which biogas can be pumped into vehicles that use biogas as fuel. The distribution station building costs would be amortized in quite a short time, if surplus biogas produced on the farms could be sold at market price. An individual biogas producer can however not trust that people driving gas vehicles would find his distribution station and buy the biogas he has produced. The uncertainty regarding the realization of biogas sales targets prevents many from making the decision to invest in a biogas facility and a biogas distribution station to be built in connection with it. At the same time, the small number of distribution stations for gas to be used as fuel limits gas-fuelled vehicles from becoming more common, even though the use of gas as a vehicle fuel is, compared to petrol or diesel, less expensive and more environmentally friendly.

It is an object of the invention to present a method, a computer program, a data system and a distribution system for the distribution of a gaseous fuel from fuel production facilities to consumers, which can reduce the disadvantages related to the prior art. The objects of the invention are obtained with a method, a computer program, a data system and a distribution system, which are characterized in what is presented in the independent claims. Some advantageous embodiments of the invention are presented in the dependent claims.

### Brief summary of the invention

The invention relates to a method for the distribution of a gaseous fuel from a fuel production facility to consumers. In the method, fuel is distributed from distribution stations, which are in connection with the fuel production facility and which distribution stations are at a distance from each other. The distance between the distribution stations can be dozens, even hundreds of kilometres. In the method, the amount of gaseous fuel to be sold is determined, the information of several distribution stations in connection with the fuel production facility is stored in a database, and the information of said several distribution stations is presented to the consumers on a digital terminal. The information to be stored in the database and presented to the consumers comprises at least the location of the fuel distribution station, the price of the fuel to be sold, and the amount of fuel on sale. Advantageously, the distribution stations are presented on a digital map, which map can be read on a portable terminal used by the consumer, such as a mobile phone. With the aid of the information shown on the terminal, the consumer can find a suitable distribution station from the ones presented.

In an advantageous embodiment of the method according to the invention, the consumer is offered a possibility to reserve a batch of fuel from the distribution station selected by the consumer. The possibility to reserve fuel is important especially when it is unsure if there is enough fuel on sale at the distribution station. Advantageously in the method, the consumer is offered a possibility to pay for the reserved fuel batch in advance or to confirm the fuel batch reservation with a preauthorization made on a means of payment.

In a second advantageous embodiment of the method according to the invention, the fuel distribution station is offered a possibility to enter and change information regarding the distribution station stored in the database or to remove information regarding the distribution station from the database. Changing information regarding the distribution station, such as fuel price information, enables among others quick and flexible special offers.

In a third advantageous embodiment of the method according to the invention, the amount of fuel sold by the distribution station/distribution stations stored in the database over a selected time period is calculated and the carbon dioxide emissions generated from the use of the sold fuel is calculated. The calculated amount of carbon dioxide emissions can be utilized later, for example in emissions trading.

The computer program for the distribution of gaseous fuel to consumers from distribution stations, which are in connection with a fuel production facility and at a distance from each other, comprises program code means for determining the amount of gaseous fuel on sale, program code means for storing information regarding several gaseous fuel distribution stations in a database, which distribution stations are in connection with a fuel production facility at a distance from each other, and program code means for presenting the information regarding said several distribution stations stored in the database to consumers on a digital terminal. The information to be stored in the database and presented to the consumers comprises at least the location of the fuel distribution station, the price of the fuel to be sold, and the amount of fuel on sale.

An advantageous embodiment of the computer program according to the invention further comprises program code means for entering the location of a first point of a driving route into the computer program, program code means for searching for distribution stations close to the first point of the driving route from the database and program code means for presenting the location of distribution stations close to the first point of the driving route on the terminal screen. Advantageously, the computer program additionally comprises program code means for entering the location of at least one second point of a driving route into the computer program, program code means for searching for distribution stations between the location of the first point and the location of the second point of the driving route and program code means for presenting the location of the distribution stations between the location of the first point and the location of the second point on the terminal screen.

A second advantageous embodiment of the computer program according to the invention additionally comprises program code means for selecting the distribution station from the distribution stations shown on the terminal screen and program code means for making fuel batch or product purchase reservation from the selected distribution station. Advantageously, the computer program further comprises program code means for entering information for a means of payment into the computer program and program code means for confirming the fuel batch or product purchase reservation with a preauthorization made on the means of payment and/or for paying the reserved fuel batch or product with the means of payment. The means of payment can be a debit card or a credit card.

Still another advantageous embodiment of the computer program according to the invention additionally comprises program code means for announcing and/or changing the amount and/or price of fuel on sale at a distribution station entered into the database. Advantageously, the embodiment further comprises program code means for entering information regarding a distribution station into the database and/or for removing it from the database. This embodiment of the computer program is intended especially for use by distribution station owners and parties operating a distribution station.

Still another advantageous embodiment of the invention additionally comprises program code means for calculating the amount of fuel sold by a distribution station/distribution stations stored in the database over a selected time period and program code means for calculating the carbon dioxide emissions generated by the use of the sold fuel.

The data system for organizing the distribution of gaseous fuel comprises a database for storing information regarding fuel distribution stations, which information includes at least information regarding the location of the distribution station, the price of the fuel being sold and/or the amount of fuel on sale, and a number of terminals, which terminals have a processor and a memory. A computer program as described above has been loaded into the memory of said terminals.

One advantageous embodiment of the data system according to the invention additionally comprises at least one server, in the memory of which said database is loaded, and said terminals can be connected with a wireless data transfer connection to said server. Advantageously, said terminals are mobile phones, which are connected to the server via a mobile phone network.

The distribution system for gaseous fuel, which includes a number of fuel production facilities and a number of fuel distribution stations, at least part of which fuel distribution stations are in connection with a fuel production facility, additionally comprises a data system as described above. Advantageously, at least a part of the fuel production facilities are biogas facilities in connection with a farm.

An advantage of the invention is that it increases the distribution network for gaseous fuel and thus improves the usability of vehicles using gas as fuel.

An additional advantage of the invention is that it improves profitability of biogas production facilities and significantly shortens the repayment period of biogas facility investments.

A further advantage of the invention is that it reduces environmental load by promoting use of gas, especially biogas, as fuel for vehicles instead of petrol or diesel.

### Brief description of the drawings

In the following, the invention will be described in detail. In the description, reference is made to the enclosed drawings, in which
figure 1 shows as an example a data system according to the invention as a simplified diagram and
figure 2 shows as an example the view on a screen of a terminal belonging to a data system according to the invention.

### Detailed description of the invention

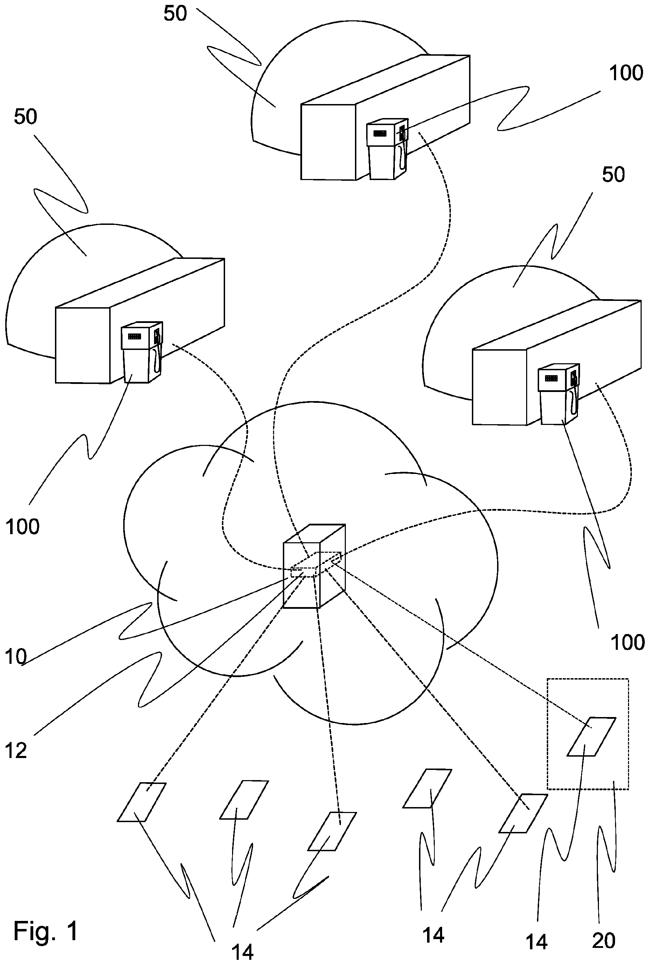

Figure 1 shows as an example a data system according to the invention as a simplified diagram. The data system includes a server 10, a so-called server computer, in the memory of which the database 12 has been loaded. The data system additionally includes a number of digital terminals 14, such as mobile phones, which can be connected to the server via a data transfer connection. The server can be a so-called cloud server, whereby the physical server computer is situated in the service provider's premises and the terminals are connected to the server via a wireless data transfer connection, advantageously a mobile phone network. The wireless data transfer connection can operate according to a GSM, GPRS, 4G, 5G or WLAN standard.

Information regarding gaseous fuel distribution stations 100 have been stored in the database in the terminal, especially such distribution stations, which are in connection with a biogas, i.e. biomethane, production facility 50. A typical biogas production facility 50 is a biogas facility in connection with a farm or a biowaste collection facility. The biogas production facility can also be a landfill, wherein a biogas collection apparatus has been built. In the database has been stored, for each fuel distribution station, at least location data for the distribution station, on the basis of which the distribution station can be placed on a digital map. The location data can be stored in the database as address information or GPS coordinates or both. Price information for the gaseous fuel being sold and amount of fuel on sale at the distribution station can additionally be stored in the database. The amount of fuel on sale can be presented as a precise number for example as kilograms, or it can be presented as a minimal value of fuel on sale, e.g. at least 1000 kg. The amount of fuel on sale can be determined by calculation, when the amount of fuel ready at the production facility, the amount of fuel needed by the production facility or distribution station for their own use and the production speed of new fuel is known. Contact information for the distribution station, such as a phone number and email address, can also be stored in the database.

Distribution stations can also have other products on sale than fuel. Especially distribution stations in connection with farms can also engage in direct sales of farm products. Also information about other products on sale at the distribution stations with prices can be stored in the database.

The distribution station 100 owner or fuel distributor authorized by the owner has access to the database 12 by means of the computer program belonging to the data system. By means of the computer program, the owner or operator can enter into the database information regarding the distribution station and update them as needed. For example information on the price and amount of fuel on sale can be updated in real time to correspond to the current situation of the distribution station. The data system thus enables for example quick special offers for fuel. Naturally, the owner or distributor can also remove information regarding the distribution station from the database or prevent their publication temporarily.

The distribution station customers, i.e. consumers, are typically owners or users of gas-driven vehicles. The consumers have access to the data system database 12 by means of a computer program to be loaded onto a digital terminal 14. Typically, the digital terminal is a mobile phone in use by the consumer and the computer program comprises an application loaded onto the mobile phone, with which a connection is formed to the database via a mobile phone network and/or some other wireless data transfer network supported by the terminal.

The data system according to the invention is part of the distribution system for gaseous fuel, which includes a number of fuel production facilities 50 and a number of fuel distribution stations 100, at least part of which fuel distribution stations are in connection with a fuel production facility. The data system is managed by an operator 20, who has a connection to the database 12 by means of his own terminal 14.

The consumers, distributor and operator are in connection with the database via in principle the same computer program. Different versions of the computer program have however been constructed, so that different user groups have different access rights to the database. The consumer versions meant for consumers only enables browsing information regarding distribution stations stored in the database and buying fuel batches or products sold at distribution stations or making a purchase reservation. The distributor version meant for fuel distribution station owners and/or distributors enables, in addition to the properties above, entering information about the distributor's own distribution station into the database, changing the entered information and removing information from the database. At least the consumer version of the computer program can be realized as an application that can be loaded onto a mobile phone.

The largest access rights are naturally given to the data system operator, who can, in addition to the properties above, add, remove and change all the information entered into the database. The operator can thus on his own terminal manage the entire data system, for example decide on what terms a distribution station is accepted into the database and, if necessary, remove distribution stations that break the terms from the database. The operator can from the database obtain information about all the fuel sold via the data system, which among others enables the calculation of carbon dioxide emissions generated through the use of the sold fuel. This information can be used for example in emissions trading.

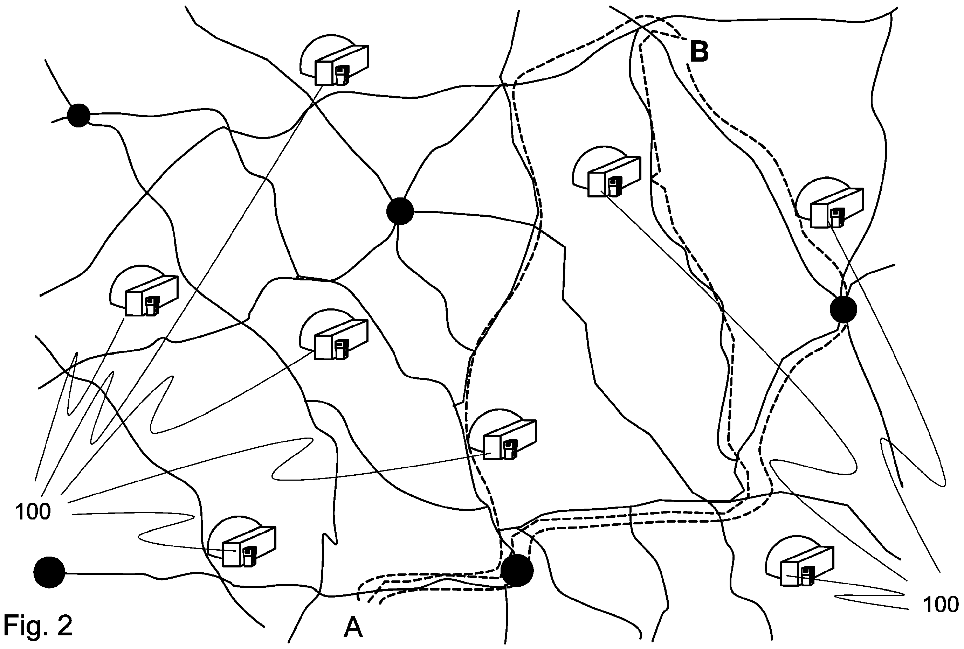

Figure 2 shows as an example the view on a screen of a terminal belonging to a data system according to the invention. Figure 2 shows the view on the screen on the terminal of a consumer, a customer searching for a fuel distribution station 100.

The consumer searching for a fuel distribution station opens the application and enters into the application the location A of the first point of the driving route. Usually, the first point of the driving route is the current location of the consumer, whereby the location can be determined with the aid of a satellite positioning software in the terminal. The location can also be entered into the application as a written address information. The location B of a second point of the driving route can also be entered into the application, which point is typically the end point or an intermediate point of the planned trip. The opened application shows the location of the entered first point of the driving route and the possibly entered second point of the driving route on the digital map shown on the screen. At the same time, the application shows on the terminal screen the fuel distribution stations 100 in the map area which are stored in the database 12. The consumer can handle the map seen on the terminal in known manners, either by scrolling the map in a desired direction, whereby the midpoint of the map visible on the screen changes, or by changing the scale of the map, whereby the geographical area visible on the screen becomes larger or smaller. When the map shown on the screen is changed, the number of fuel distribution stations shown on the screen correspondingly changes.

When the consumer has entered the location A of the first point of the driving route and the location B of the second point of the driving route into the application, a map is thus revealed on the terminal screen, where the points entered by the consumer are shown and all the distribution stations 100 entered into the data system database, which are located in the same geographical area. The conurbations in the map shown in Figure 2 are marked with black dots and the roads with black continuous lines. Typically, farms and distribution stations for gaseous fuel in connection with them are located outside conurbation centres. The price of the fuel on sale can also be shown in the view of Figure 2 by the icon illustrating each distribution station 100. In order to preserve the clarity of the view, the fuel price and the amount of fuel on sale can be presented in a separate window, which opens, when the pointer of the terminal is brought onto the icon illustrating the distribution station or when the icon illustrating the distribution station is clicked with the pointer of the terminal.

With the aid of the information shown on the terminal screen, the consumer can select a suitable fuel distribution station 100 on their driving route. When making the selection, the consumer can review different alternative driving routes leading from the first point A to the second point B and compare the price and amount information for fuel in the distribution stations along these driving routes. In Figure 2 is shown as an example with dotted lines three alternative driving routes from the first point A to the second point B.

When the consumer has selected a desired distribution station, he can, if so desired, reserve a desired fuel batch from the selected distribution station 100. In order to make the reservation, information about a means of payment to be used, i.e. the information regarding a debit card or credit card, and the size of the reserved fuel batch, is entered into the application on the terminal. The application gives the consumer a confirmation regarding the made reservation and a preauthorization of the reservation is made on the means of payment. At the same time, the information regarding the amount of fuel on sale at the distribution station in question is correspondingly updated. The possibility to reserve a fuel batch is especially important in areas, where the distribution stations are far from each other and the amount of fuel on sale at the distribution station varies widely. By reserving a batch of fuel, the consumer ensures that the fuel batch he reserved is for sure available at the distribution station.

Some advantageous embodiments of the method and computer system according to the invention have been described above. The invention is not limited to the solutions described above, but the inventive idea can be applied in different ways within the scope of the claims.

### List of reference numbers:

- 10: server
- 12: database
- 14: terminal
- 20: operator
- 50: fuel production facility
- 100: distribution station

## Claims

1. A method for distributing gaseous fuel from a fuel production facility (50) to consumers, in which method fuel is distributed from distribution stations (100), which are in connection with the fuel production facility (50) and which distribution stations (100) are at a distance from each other, **characterized in that** the method comprises determining the amount of gaseous fuel on sale, storing information regarding several distribution stations (100) in connection with the fuel production facility (50) in a database (12) and presenting information regarding said several distribution stations (100) to a consumer on a digital terminal (14), which information stored in the database and presented to the consumer comprise at least the location of the fuel distribution station (100), the price of the fuel to be sold and the amount of fuel on sale.

2. The method according to claim 1, **characterized in that** the distribution stations (100) are shown on a digital map, which map can be read on a portable terminal (14) used by the consumer, such as a mobile phone.

3. The method according to claim 1 or 2, **characterized in that** the consumer is offered a possibility to reserve a batch of fuel from the distribution station (100) selected by the consumer, to reserve and pay for the reserved fuel batch in advance or to reserve and confirm the reservation of the fuel batch with a preauthorization made on the means of payment.

4. The method according to any of the claims 1-3, **characterized in that** the fuel distribution station (100) is offered a possibility to enter and change information regarding the distribution station (100) stored in the database (12) or to remove information regarding the distribution station (100) from the database (12).

5. The method according to any of the claims 1-4, **characterized in that** the amount of fuel sold by the distribution station/distribution stations (100) stored in the database (12) over a selected time period is calculated and the carbon dioxide emissions generated from the use of the sold fuel is calculated.

6. A computer program for distributing gaseous fuel to consumers from distribution stations (100), which are in connection with a fuel production facility (50) and at a distance from each other, **characterized in that** said computer program comprises program code means for determining the amount of gaseous fuel on sale, program code means for storing information about several gaseous fuel distribution stations (100) in a database (12), which distribution stations (100) are in connection with a fuel production facility (50) at a distance from each other, and program code means for presenting information about said several distribution stations (100) stored in the database (12) to a consumer on a digital terminal (14), which information stored in the database and presented to the consumer comprises at least the location of the fuel distribution station, the price of the fuel being sold and the amount of fuel on sale.

7. The computer program according to claim 6, **characterized in that** it further comprises
- program code means for entering the location of a first point of a driving route into the computer program,
- program code means for searching for distribution stations (100) close to the first point of the driving route from the database (12) and
- program code means for presenting the location of distribution stations (100) close to the first point of the driving route on the terminal (14) screen.

8. The computer program according to claim 7, **characterized in that** the computer program further comprises
- program code means for entering the location of at least one second point of a driving route into the computer program,
- program code means for searching for distribution stations (100) between the location of the first point and the location of the second point of the driving route from the database (12) and
- program code means for presenting the location of distribution stations (100) between the location of the first point and the location of the second point on the terminal (14) screen.

9. The computer program according to claim 6-8, **characterized in that** the computer program further comprises
- program code means for selecting the distribution station (100) from the distribution stations shown on the terminal screen and
- program code means for making fuel batch or product purchase reservation from the selected distribution station (100).

10. The computer program according to claim 9, **characterized in that** the computer program further comprises
- program code means for entering information for a means of payment into the computer program and
- program code means for confirming the fuel batch or product purchase reservation with a preauthorization made on the means of payment and/or for paying the reserved fuel batch or product with the means of payment.

11. The computer program according to any of the claims 6-10, **characterized in that** it further comprises
- program code means for announcing and/or changing the amount and/or price of fuel on sale at a distribution station (100) entered into the database (12).

12. The computer program according to any of the claims 6-11, **characterized in that** it further comprises program code means for entering information regarding a distribution station (100) into the database (12) and/or removing it from the database (12).

13. The computer program according to any of the claims 6-12, **characterized in that** it further comprises program code means for calculating the amount of fuel sold by the distribution station/distribution stations (100) stored in the database (12) over a selected time period and program code means for calculating the carbon dioxide emissions generated from the use of the sold fuel.

14. A data system for organizing the distribution of gaseous fuel, which data system comprises a database (12) for storing information regarding fuel distribution stations (100), which information includes at least information regarding the location of the distribution station (100), the price of the fuel being sold and/or the amount of fuel on sale, and a number of terminals (14), which terminals (14) have a processor and a memory, **characterized in that** a computer program according to any of the claims 8-15 has been loaded into the memory of said terminals (14).

15. The data system according to claim 14, **characterized in that** the data system additionally comprises at least one server (10), in the memory of which said database (12) is loaded, and said terminals (14) are mobile phones, which are connected to the server (10) via a mobile phone network.

16. A distribution system for gaseous fuel, which includes a number of fuel production facilities (50) and a number of fuel distribution stations (100), at least part of which fuel distribution stations (100) are in connection with a fuel production facility (50), **characterized in that** the distribution system additionally comprises a data system according to claim 14 or 15.

17. The fuel distribution system according to claim 16, **characterized in that** at least part of the fuel production facilities (50) are biogas facilities in connection with a farm.
